# EUROPÄISCHE PATENTSCHRIFT

(11) **EP 1 226 829 B1**
(45) Veröffentlichungstag und Bekanntmachung des Hinweises auf die Patenterteilung: **11.06.2008**
(21) Anmeldenummer: 01129605.0
(22) Anmeldetag: 12.12.2001
(51) Int. Cl.: A61K 38/48, A61K 47/18

(54) **Stabilisierte Flüssigzubereitung der den Blutgerinnungsfaktor VII aktivierenden Protease oder ihres Proenzyms**
Stabilised liquid preparations of the protease or its proenzyme which activitate the blood coagulation factor VII
Préparations liquides stabilisées de la protéase ou de son proenzyme qui activent le facteur VII de la coagulation sanguine

(30) Priorität: 08.01.2001 DE 10100483; 25.06.2001 DE 10131404
(43) Veröffentlichungstag der Anmeldung: 31.07.2002
(73) Patentinhaber: CSL Behring GmbH, 35041 Marburg (DE)
(72) Erfinder: Roemisch, Jürgen, 35041 Marburg (DE); Feussner, Annette, 35043 Marburg (DE); Kannemeier, Christian, 35390 Giessen (DE); Stoehr, Hans-Arnold, 35083 Wetter (DE)

(56) Entgegenhaltungen:
- EP-A- 0 952 215
- EP-A- 1 074 615
- EP-A- 1 153 608

## Beschreibung

Gegenstand der Erfindung ist eine stabilisierte Flüssigzubereitung der den Blutgerinnungsfaktor VII aktivierenden Protease oder ihres Proenzyms, die über mehrere Monate lagerfähig ist, ohne wesentliche Aktivitätseinbußen oder Produktveränderungen zu zeigen.

Aus der deutschen Offenlegungsschrift 199 03 693 sind eine Protease zur Aktivierung des Blutgerinnungsfaktors VII, ein Verfahren zu ihrer Gewinnung, zu ihrem Nachweis und zu ihrer Inaktivierung sowie Arzneizubereitungen bekannt, die diese Protease oder ihr Proenzym enthalten. Dort wird auch berichtet, dass diese Protease und ihr Proenzym nach Anreicherung oder Isolierung einen raschen Aktivitätsverlust erleiden, der in einer Lösung enthaltend 20 mM Tris, 0,15 M NaCI bei einem pH-Wert von 7,5 beobachtet wurde. Zur Stabilisierung wurde dort eine Zubereitung vorgeschlagen, die
a) unter Zusatz einer oder mehrerer Aminosäuren in einer Menge von 0,01 mol/l, vorzugsweise mehr als 0,05 mol/l; und/oder
b) unter Zusatz eines Zuckers oder einer Kombination verschiedener Zucker mit einer Gesamtkonzentration von mehr als 0,05 g/ml, vorzugsweise mehr als 0,2 g/ml und/oder
c) unter Zusatz einer oder mehrerer Substanzen, die Calciumionen zu komplexieren vermögen, wie Zitrat, Oxalat, Ethylendiamintetraessigsäure usw. hergestellt wird.

Dabei wird die Zubereitung auf einen pH-Bereich von 3,5 bis 8,0, vorzugsweise auf einen pH-Bereich von 4,0 bis 6,8 eingestellt.

Obwohl durch die Anwendung der vorstehend genannten Mittel bereits beachtliche Stabilisierungserfolge erreicht werden, hat es sich als notwendig erwiesen, nach weiteren Stabilisatoren und Stabilisatorgemischen zu suchen, die für den Einsatz in Flüssigzubereitungen der genannten Protease und ihres Proenzyms geeignet sind und sicherstellen, dass derartige Zubereitungen über längere Zeiträume, die mehrere Monate betragen können, ohne wesentliche Aktivitätseinbußen oder sonstige Produktveränderungen gelagert werden können.

Es wurde nun gefunden, dass eine Flüssigzubereitung der den Blutgerinnungsfaktor VII aktivierenden Protease oder ihres Proenzyms für längere Zeiträume, d.h. über mehrere Monate, stabilisiert ist, wenn sie eine oder mehrere Verbindungen ausgewählt der Gruppe Ornithin, Diaminopimelinsäure, Agmatin, Kreatin, Guanidinoessigsäure, Acetylornithin, Citrullin, Argininobernsteinsäure, Tranexamsäure und ε-Aminocapronsäure oder eines ihrer Salze und Derivate enthält und einen pH-Wert zwischen 2,0 und 8,0 aufweist, vorzugsweise 2,5 bis 6,8. Auch ein pH Bereich von 3,5 bis 6,8 ist gut anwendbar.

Die Stabilität einer derartigen Zubereitung kann noch weiter verbessert werden, wenn sie zusätzlich
a) ein oder mehrere Detergentien;
b) einen oder mehrere Zucker;
c) eine oder mehrere Aminosäuren; und/oder
d) Proteine, vorzugsweise Albumin, Gelatine, Fibronectin und Vitronektin oder ähnliche Proteine, und/oder
e) eine oder mehrere Verbindungen, die Calciumionen zu komplexieren vermögen, enthält.

Als Detergentien werden ein oder mehrere ionische oder nicht-ionische Tenside eingesetzt, die zum Beispiel unter dem Handelsnamen Tween® und Triton® bekannt sind. Sie werden im allgemeinen in Konzentrationen zwischen 0,001 und 0,5 Gewichtsprozent eingesetzt.

Die Protease oder ihr Proenzym wird erfindungsgemäß in lyophilsierter Form verwendet. Nach Lösen des Lyophilisats bleibt die Aktivität im Vergleich zur Lösung vor der Gefriertrocknung zu mehr als 90% erhalten. Besonders gut lassen sich die Protease und ihr Proenzym in Lösung halten, wenn die lonenstärke der Lösung von größer als >10 mSi erreicht wird. Hierzu muss eine ausreichende Menge eines Salzes, zum Beispiel Kochsalz, zugesetzt werden. Die lonenstärke ist besonders dann von großer Bedeutung, wenn Proteasekonzentrationen von über 0,5 mg/ml verwendet werden.

Unter den erfindungsgemäß zur Stabilisierung zu verwendenden Zuckern sind Glukose, Arabinose oder Mannose vor allem zu nennen. Sie können in Mengen von 5 bis 100 mM eingesetzt werden.

Als Aminosäuren können der Flüssigzubereitung vor allem Arginin, Lysin oder Glyzin zugesetzt werden. Als Calciumionen komplexierende Verbindungen kommen neben Zitraten auch Oxalate und Salze der Ethylendiamintetraessigsäure in Betracht.

Die so hergestellten Flüssigzubereitungen der den Blutgerinnungsfaktor VII aktivierenden Protease oder ihres Proenzyms sind zur Durchführung einer Pasteurisierung, einer Nanofiltration oder einer Dampf/Hitze-Virusinaktivierung geeignet. Sie können als Blutgerinnungsmittel entweder allein oder zusammen mit die Proteaseaktivität erhöhenden Substanzen wie Heparin oder dem Heparin verwandten Substanzen wie Heparansulfat eingesetzt werden, wobei diesen Mitteln zusätzlich noch der Faktor VII in seiner inaktiven Form zugesetzt sein kann. Die Anwendung eines derartigen Mittels kann zum Beispiel unter Ausnutzung seiner FVIII bypassing aktivity (FEIBA) angezeigt sein, wenn Unverträglichkeiten gegenüber dem Faktor VIII und/oder Faktor IX und/oder Faktor XI und/oder den Proteinen der Kontaktphase, wie Faktor XII zum Beispiel wegen des Vorliegens von Antikörpern bestehen, oder andersartige Mangelsituationen vorliegen. Die Aktivierung des Faktor VIII kann dann entweder in vivo, in Plasma, in angereicherten Fraktionen oder durch Einwirkung auf gereinigten Faktor VII erfolgen. Auch die Anwendung ex vivo zur allgemeinen Blutungsprophylaxe oder zur Stillung von Blutungen kann mit der erfindungsgemäßen Flüssigzubereitung durchgeführt werden.

Die erfindungsgemäßen Flüssigzubereitungen können aber auch bei thromboembolischen Erkrankungen oder Komplikationen wie bei der Beinvenenthrombose, dem Herzinfarkt oder Schlaganfällen eingesetzt werden.

Aus der deutschen Offenlegungsschrift 199 03 693 ist bekannt, dass die den Blutgerinnungsfaktor VII aktivierende Protease zur endogenen oder exogenen Aktivierung von Plasminogenaktivatoren wie der Prourokinase oder des sctPAs verwendet werden kann. Aufgrund dieser Eigenschaft kann die den Faktor VII aktivierende Protease zur Prophylaxe oder Therapie von thromboembolischen Erkrankungen eingesetzt werden, und zwar auch in Kombination mit Einketten- oder Zweiketten-Plasminogenaktivatoren oder Antikoagulantien. Die die Plasminogenaktivatoren verstärkende Wirkung des Faktor VII-Aktivators wird besonders durch Kalzium und/oder Heparin und heparinähnliche Substanzen wie Dextransulfat gefördert. Aufgrund der besonderen fibrinolytischen Wirkung können die erfindungsgemäßen Zubereitungen, die die den Blutgerinnungsfaktor VII aktivierende Protease enthalten, zur Behandlung von Erkrankungen eingesetzt werden, die durch fibrinhaltige Thromben verursacht werden. Hierzu gehören auch Wundheilungsprozesse. Dabei kann die genannte Protease intravenös oder lokal, subkutan, intradermal, intramuskulär oder bei Verletzungen oder Wunden auch topisch oder gebunden an eine geeignete Trägermatrix verabreicht werden. Nicht nur die aus Körperflüssigkeiten wie Blut oder Plasma gewonnene Protease oder ihr Proenzym, sondern auch rekombinant oder transgen hergestellte Protease kann dafür eingesetzt werden.

## Patentansprüche

1. Stabilisierte Flüssigzubereitung der den Blutgerinnungsfaktor VII aktivierenden Protease oder ihres Proenzyms, **dadurch gekennzeichnet, dass** sie
a) eine oder mehrere Verbindungen ausgewählt aus der Gruppe Ornithin, Diaminopimelinsäure, Agmatin, Kreatin, Guanidinoessigsäure, Acetylomithin, Citrullin, Argininobemsteinsäure, Tranexamsäure und ε-Aminocapronsäure oder ihrer Salze und Derivate enthält und
b) einen pH-Wert zwischen 2,0 und 8,0 aufweist.

2. Stabilisierte Flüssigzubereitung nach Anspruch 1, **dadurch gekennzeichnet, dass** sie zusätzlich
a) ein oder mehrere Detergentien;
b) einen oder mehrere Zucker;
c) eine oder mehrere Aminosäuren; und/oder
d) eine oder mehrere Verbindungen, die Calciumionen zu komplexieren vermögen, enthält.

3. Stabilisierte Flüssigzubereitung nach den Ansprüchen 1 und 2, **dadurch gekennzeichnet, dass** sie ein oder mehrere ionische oder nicht-ionische Detergentien enthält.

4. Stabilisierte Flüssigzubereitung nach den Ansprüchen 1 bis 3, **dadurch gekennzeichnet, dass** sie die Detergentien in einer Menge von 0,001 bis 0,5 Gewichtsprozent enthält.

5. Stabilisierte Flüssigzubereitung nach den Ansprüchen 1 bis 4, **dadurch gekennzeichnet, dass** sie durch Salzzugabe auf eine lonenstärke von >10 mSi eingestellt ist.

6. Stabilisierte Flüssigzubereitung nach den Ansprüchen 1 bis 5, **dadurch gekennzeichnet, dass** sie die Protease oder ihr Proenzym in lyophilisierter Form enthält.

7. Stabilisierte Flüssigzubereitung nach den Ansprüchen 1 bis 6, **dadurch gekennzeichnet, dass** die Protease oder ihr Proenzym in einer Menge von >0,5 mg/ml vorliegt.

8. Verwendung einer Flüssigkeit nach Anspruch 1 zur Herstellung einer pharmazeutischen Zusammensetzung.

9. Verwendung einer Flüssigkeit nach Anspruch 1 zur Herstellung eines diagnostischen Reagenz.

## Claims

1. Stabilized liquid preparation of the protease which activates blood coagulation factor VII or of its proenzyme, which
a) comprises one or more compounds selected from the group of ornithine, diaminopimelic acid, agmatine, creatine, guanidinoacetic acid, acetylornithine, citrulline, argininosuccinic acid, tranexamic acid and ε-aminocaproic acid or their salts and derivatives, and
b) has a pH between 2.0 and 8.0.

2. Stabilized liquid preparation according to Claim 1, which additionally comprises
a) one or more detergents;
b) one or more sugars;
c) one or more amino acids; and/or
d) one or more compounds capable of calcium ion complexation.

3. Stabilized liquid preparation according to Claims 1 and 2, which comprises one or more ionic or nonionic detergents.

4. Stabilized liquid preparation according to Claims 1 to 3, which comprises the detergents in an amount of from 0.001 to 0.5 percent by weight.

5. Stabilized liquid preparation according to Claims 1 to 4, which is adjusted to an ionic strength of > 10 mSi by addition of salt.

6. Stabilized liquid preparation according to Claims 1 to 5, which comprises the protease or its proenzyme in lyophilized form.

7. Stabilized liquid preparation according to Claims 1 to 6, **characterized in that** the protease or its proenzyme is present in an amount of > 0.5 mg/ml.

8. Use of a liquid according to Claim 1 for preparing a pharmaceutical composition.

9. Use of a liquid according to Claim 1 for preparing a diagnostic reagent.

## Revendications

1. Préparation liquide stabilisée de la protéase activant le facteur de coagulation VII, ou de la proenzyme de cette protéase, **caractérisée en ce qu'**elle
a) contient un ou plusieurs composés choisis dans le groupe constitué par l'ornithine, l'acide diaminopimélique, l'agmatine, la créatine, l'acide guanidinoacétique, l'acétylornithine, la citrulline, l'acide argininosuccinique, l'acide tranexamique et l'acide ε-aminocaproïque ou leurs sels et dérivés et
b) présente un pH compris entre 2,0 et 8,0.

2. Préparation liquide stabilisée selon la revendication 1, **caractérisée en ce qu'**elle contient en outre
a) un ou plusieurs détergents,
b) un ou plusieurs sucres,
c) un ou plusieurs acides aminés, et/ou
d) un ou plusieurs composés pouvant complexer les ions calcium.

3. Préparation liquide stabilisée selon les revendications 1 et 2, **caractérisée en ce qu'**elle contient un ou plusieurs détergents ioniques ou non ioniques.

4. Préparation liquide stabilisée selon les revendications 1 à 3, **caractérisée en ce qu'**elle contient des détergents en une quantité de 0,001 à 0,5 % en poids.

5. Préparation liquide stabilisée selon les revendications 1 à 4, **caractérisée en ce qu'**elle est ajustée à une force ionique de >10 mSi par addition de sels.

6. Préparation liquide stabilisée selon les revendications 1 à 5, **caractérisée en ce qu'**elle contient la protéase ou sa proenzyme sous forme lyophilisée.

7. Préparation liquide stabilisée selon les revendications 1 à 6, **caractérisée en ce que** la protéase ou sa proenzyme est présente en une quantité de >0,5 mg/ml.

8. Utilisation d'un liquide selon la revendication 1, pour la préparation d'une composition pharmaceutique.

9. Utilisation d'un liquide selon la revendication 1, pour la préparation d'un réactif de diagnostic.
